# EUROPÄISCHE PATENTANMELDUNG

(11) **EP 1 698 328 A2**
(43) Veröffentlichungstag der Anmeldung: **06.09.2006**
(21) Anmeldenummer: 06002865.1
(22) Anmeldetag: 23.11.2002
(51) Int. Cl.: A61K 9/107, A61Q 5/00, A61Q 19/00, A61K 31/355, A23L 1/302

(54) **Tocopherol-Konzentrat**

(30) Priorität: 30.11.2001 DE 10158447
(62) Teilanmeldung aus: 02026130.1
(71) Anmelder: AQUANOVA German Solubilisate Technologies (AGT) GmbH, 64295 Darmstadt (DE)
(72) Erfinder: Behnam, Dariush, 64380 Roßdorf (DE)
(74) Vertreter: Blumbach - Zinngrebe

(57) **Zusammenfassung**

Beschrieben wird ein Tocopherol-Konzentrat, welches aus einem Solubilisat aus 10 Gew% eines Mischtocopherols und 90 Gew% Polysorbat 20 besteht.

## Beschreibung

Die Erfindung betrifft eine wässrige Lösung von Ascorbinsäure.

Die technische, insbesondere die körperpflegemittel- und lebensmitteltechnische Verwendung und prophylaktische Anwendung des Reduktons Ascorbinsäure wird oft dadurch erschwert, daß die Ascorbinsäure in wässriger Lösung nicht ausreichend beständig ist. So sind in einer wässrigen Ascorbinsäurelösung schon nach etwa 30 Tagen nur noch etwa 70% der zu zugestzten Ascorbinsäure vorhanden.

Man hat die Beständigkeit dadurch zu verbessern versucht, daß man statt Ascorbinsäure eines ihrer Derivate, beispielsweise Natriumascorbinphosphat oder Ascorbylpalmitat eingesetzt hat. Der Gehalt dieses Wirkstoffes bleibt in wässriger Lösung zwar über lange Zeit nahezu erhalten. Jedoch ist das Derivat um ein Vielfaches teurer als reine Ascorbinsäure. Außerdem neigen die Ascorbinsäurederivate bei höherer Konzentration zum Auskristallisieren und führen zu einer Einfärbung des Endproduktes.

In der europäischen Patentschrift 660676 ist eine Zusammensetzung bestehend aus 0,1-2,0 Gew% eines öllöslichen Bestandteils, der bevorzugt ein farbgebendes Carotenoid ist, aus 2-20 Gew% eines Emulgators mit einem HLB-Wert von 10-18 und aus 0,1-1,0Gew% eines Antoxidationsmittels bekannt. Der Emulgator kann Polysorbat 40 oder 60 sein, als Antioxidationsmittel wird unter anderem Ascorbinsäure empfohlen. Damit sollen farblich klare und stabile Getränke für die Gesundheitsvorsorge herstellbar sein. Ungeklärt bleibt jedoch die Stabilität der Ascorbinsäure im Endprodukt.

Der Erfindung liegt demgegenüber die Aufgabe zugrunde, Ascorbinsäure in flüssiger, wasser- und fettlöslicher und in verkapselbarer Form (z.B. Gelatinekapsel) bereitzustellen, die über viele Monate hin beständig ist und in hoher Konzentration vorliegt.

Diese Aufgabe wird nach Erfindung dadurch gelöst, daß eine wässrige Lösung von Ascorbinsäure einen Überschuß an einem Emulgator mit einem HLB-Wert von 9 - 18 wie etwa ein Polysorbat, vorzugsweise Polysorbat 80 enthält. Die Lösung ist bei Raumtemperatur klar und fast gelartig, trübungsfrei mit Wasser verdünnbar und läßt sich bei Erwärmung auf ungefähr 35°C mit wässrigen oder fettigen Endprodukten aus dem Kosmetik- oder Lebensmittelbereich ohne weitere Verarbeitungsschritte mühelos homogen vermischen. Der Ascorbinsäureanteil der erfindungsgemäßen Lösung, der bis zu etwa 20 Gew% betragen kann, bleibt über wenigstens ein halbes Jahr praktisch verlustfrei erhalten. Der Polysorbatanteil beträgt zweckmäßig etwa 60 Gew%, der Rest ist Wasser. Das erfindungsgemäße Ascorbinsäuresolubilisat läßt sich leicht Kosmetika (Haut- und Haarpflegemitteln), Lebensmitteln, Arzneimitteln und Nährlösungen zur Züchtung von Zell- und Bakterienkulturen sowie von Algen (Mikroalgen) mit der Folge zusetzen, daß die Beständigkeit dieser Produkte wesentlich erhöht ist.

Die Ascorbinsäure liegt in dem Solubilisat micelliert vor. Eine elektronenmikroskopische Untersuchung eines 10%igen Ascorbinsäuresolubilisats in einer Verdünnung von 1:1000 zeigt einen Micellendurchmesser von etwa 100 nm. Da die Emulgatorhülle der Micellen die eingeschlossene Ascorbinsäure nur verzögert (retardierend) freigibt, bleibt die antioxydative Wirkung des erfindungsgemäßen Solubilisats in den erwähnten Mitteln in Kombination mit solubilisierten Misch-Tocopherolen länger wirksam als bei Zusatz etwa von Ascorbylpalmitat, also einem der eingangs genannten Ascorbinsäurederivate.

Ernährungsphysiologisch verhindert die Micellierung der Ascorbinsäure durch den Emulgator, daß bei oraler Gabe des erfindungsgemäßen Solubilisats die Ascorbinsäure bereits im mittleren Verdauungstrakt, also im Magen und Duodenum ihre Wirkung entfaltet und sich dabei verbraucht. Die micellierte Ascorbinsäure wird vielmehr erst im Dünndarm resorbiert.

In besonders bevorzugter Ausgestaltung der Erfindung enthält das Ascorbinsäuresolubilisat einen Zusatz an Tocopherol, insbesondere an einem Misch-Tocopherol. Durch Zusatz dieses Ascorbinsäure-Tocopherol-Solubilisats zu organischen Ölen, z.B. Pflanzenölen wie etwa Sonnenblumenöl, Distelöl, Leinöl und dergleichen kann deren Haltbarkeit ganz wesentlich verbessert werden. Als Tocopherol kommt entweder reines α-Tocopherol oder besonders bevorzugt eine Mischung aus α-, β-, γ- und δ-Tocopherol in Betracht. Zu empfehlen ist ein Misch-Tocopherol, welches etwa 8,0 bis etwa 20,0 Gew% an α-Tocopherol, etwa 1,5 bis etwa 4,5 Gew% an β-Tocopherol, etwa 55,0 bis etwa 70,0 Gew% an γ-Tocopherol und etwa 15,0 bis etwa 27,0 Gew% an δ-Tocopherol enthält.

Wenn der erfindungsgemäßen Lösung eine Octadecatriensäure und/oder eine Octadecensäure, etwa in Form von α-Linolensäure, γ-Linolensäure, Linolsäure oder Ölsäure, zugesetzt ist, ist die Viskosität der Lösung herabgesetzt. Sie ist bei Raumtemperatur klar, zähflüssig, mit Wasser trübungsfrei verdünnbar bzw. mit wässrigen und/oder fettigen Nahrungsmitteln, Kosmetika und Pharmazeutika ohne weitere Verarbeitungsschritte vermischbar. Der Polysorbatgehalt der erfindungsgemäßen Lösung entfaltet eine Art Retardfunktion für die konservative Eigenschaft der Ascorbinsäure für die im allgemeinen leicht oxydierbaren Inhaltsstoffe von Salben und dergleichen Zubereitungen. Damit bleibt die erwünschte Schutzfunktion der Ascorbinsäure über einen längeren Zeitraum erhalten. Der Ascorbinsäuregehalt der Lösung kann zweckmäßig bei etwa 5 Gew% bis etwa 15 Gew% liegen. Die Lösung kann vorteilhaft etwa 10 Gew% bis etwa 20 Gew% an Octadecatriensäure und/oder Octadecensäure enthalten. Der Polysorbatanteil beträgt vorzugsweise etwa 60 Gew% bis etwa 75 Gew%.

Wird der Wasseranteil der erfindungsgemäßen Lösung auf etwa 5 Gew% bis etwa 7 Gew% herabgesetzt und der Polysorbatanteil entsprechend erhöht, kann die bei Raumtemperatur zähflüssige Lösung auch Wirkstoffen beigemischt werden, die anschließend in Gelatinekapseln oder gelatinefreie Kapseln abgefüllt werden. Der geringe Wasseranteil der Lösung läßt die Kapselhülle unbeschädigt, wobei die Schutzfunktion der Ascorbinsäure für den Wirkstoff unbeeinträchtigt bleibt.

Ein Verfahren zur Herstellung der erfindungsgemäßen Lösung sieht vor, daß einer wässrigen Ascorbinsäurelösung ein Emulgator mit einem HLB-Wert von etwa 9 bis etwa 18, beispielsweise ein Polysorbat, zweckmäßig Polysorbat 80, zugegeben und das Gemisch unter Rühren bis zur Klarheit und Homogenität kurzzeitig aufgewärmt wird. Die in der Lösung sich bildenden Micellen mit einem Durchmesser von etwa 100 nm weisen eine doppelwandige Hülle aus radial orientierten Polysorbatmolekülen auf, wobei die Polysorbatmoleküle der Innenhülle mit ihren hydrophilen Abschnitten auf die Ascorbinsäurelösung ausgerichtet sind und die hydrophilen Abschnitte der Polysorbatmoleküle der Außenhülle sich außen anordnen. Die auf diese Weise gewonnene Lösung ist in Wasser trübungslos verdünnbar und läßt sich technisch ohne weitere Verarbeitungsschritte einsetzen.

Die Beständigkeit der Micellen wird erhöht, wenn in Weiterbildung der Erfindung der wässrigen Ascorbinsäurelösung ein im wesentlichen aus Triglyceriden bestehendes leichtes natürliches Öl, wie etwa Distelöl, zusammen mit dem Emulgator zugesetzt wird. Die sich dabei bildenden Ölmicellen adhärieren an den Ascorbinsäuremicellen und schützen diese.

Zweckmäßig wird der Lösung nach leichtem Erwärmen auf etwa 50°C unter Rühren eine Octadecatriensäure und/oder eine Octadecensäure etwa in Form von α-Linolensäure, γ-Linolensäure, Linolsäure oder Ölsäure vor der Zugabe von Polysorbat zugesetzt. Für die Befüllung von Kapseln, deren Hülle aus Gelatine bestehen oder die gelatinefrei sein kann, ist es vorteilhaft, wenn Ascorbinsäure in der gleichen Menge aqua dest gelöst, der Lösung die gleiche bis maximal doppelte Menge wie Ascorbinsäure an Fettsäuren unter leichter Erwärmung zugegeben und die etwa zwei- bis etwa dreifache Menge an Polysorbat 80 zugesetzt und das Gemisch auf etwa 80°C erwärmt und gerührt wird.

Die nachstehenden Ausführungsbeispiele verdeutlichen die Erfindung.

### Beispiel 1

20g Ascorbinsäure werden in 20g destilliertem, entgasten Wasser vollständig aufgelöst. Der Lösungsvorgang kann beschleunigt werden, wenn das Wasser auf etwa 45 °C erwärmt wird. Anschließend werden der Lösung 60g Polysorbat 80 unter Rühren und Aufwärmen auf etwa 80°C zugegeben. Es wird gerührt bis zur Klarheit und Homogenität des Solubilisats, das bei Raumtemperatur klar und fast gelartig erscheint, in Wasser trübungslos verdünnbar ist und sich ohne weitere Verarbeitungsschritte den zu konservierenden Zusammensetzungen oder Nahrungsmitteln, Getränken, Kosmetika und Pharmazeutika zusetzen läßt.

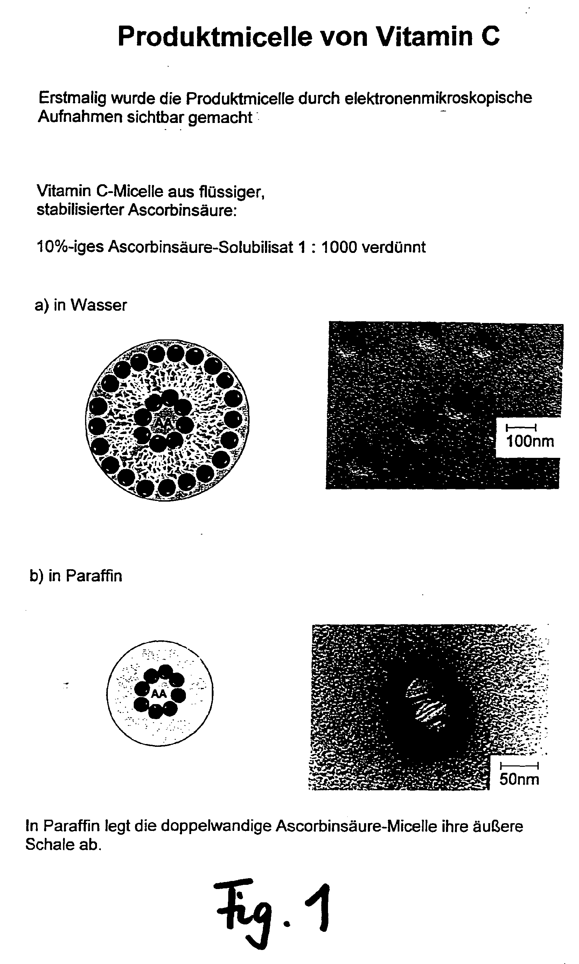

Für ein geringer konzentriertes Solubilisat löst man 10g Ascorbinsäure in 10g aqua dest auf und gibt 80g Polysorbat 80 hinzu, verfährt im übrigen wie vorstehend. Von dem daraus erhaltenen 10%igen Ascorbinsäuresolubilisat wurde nach dessen wässriger Verdünnung zu 1 : 1000 eine elektronenmikroskopische Aufnahme gemacht, die in Figur 1 wiedergegeben ist. Man sieht, daß die Ascorbinsäuresolubilisatmicellen einen Durchmesser von etwa 100 nm haben. Einbringen des Solubilisats in Paraffin läßt den Micellendurchmesser auf die Hälfte schrumpfen, wie man aus der unteren elektronenmikroskopischen Aufnahme der Figur 1 erkennt. Diese Erscheinung kann man damit erklären, daß die Micellen in Paraffin die äußere Polysorbathülle verlieren.

### Beispiel 2

Wie bei Beispiel 1 löst man 10 Gew% Ascorbinsäure (bezogen auf die Gesamtmenge an Ascorbinsäuresolubilisat = 100%) in 10Gew% aqua dest und gibt zu dieser wässrigen Lösung 70 Gew% Polysorbat 80 zusammen mit 10 Gew% eines leichten Pflanzenöls wie etwa Distelöl oder Leinöl. Man erwärmt auf mindestens 60°C und rührt bis zur Klarheit und Homogenität des Solubilisats, das sich bei Raumtemperatur in Wasser klar und rückstandsfrei lösen läßt.

Reduziert man den Gehalt an Ascorbinsäure auf etwa 7 Gew% und den Wassergehalt auf ebenfalls etwa 7 Gew% und erhöht die weiteren Anteile des Solubilisats entsprechend, kann es wegen des relativ geringen Wasseranteils besonders gut als Konservierungsmittel für Wirkstoffe eingesetzt werden, die als Retardpräparate verkapselt werden sollen. Die Kapselhülle wird von dem Solubilisat praktisch nicht angegriffen, was bei einem höheren Wassergehalt zu befürchten wäre.

### Beispiel 3

10 g Ascorbinsäure werden in 10 g aqua dest. aufgelöst und der Lösung 20 g Distelöl zugegeben. Dieser Mischung werden 110 g Polysorbat 80 zugesetzt, das Ganze unter Rühren auf etwa 100°C bis zur Wasserfreiheit, d.h. bis zur Beendigung des Kochens, erhitzt. Nach Abkühlen auf Zimmertemperatur liegt ein 6,5 %iges Ascorbinsäure-Solubilat in dem Lösungsvermittler vor, dessen Wassergehalt deutlich unter 5 Vol% liegt und das sowohl in Wasser als auch in Fetten und Ölen löslich ist. Eine Prüfung des Ascorbinsäuregehalts der Lösung durch ein unabhängiges Chemisches Untersuchungslabor zum Zeitpunkt der Herstellung der Lösung und mehr als fünf Monate später ergab einen Ascorbinsäureverlust während dieser Zeit von nur etwa 3%.

### Beispiel 4

Man geht aus von dem Solubilisat nach Beispiel 2. Diesem wird ein Misch-Tocopherol-Solubilisat zugegeben, das auf folgende Weise gewonnen wird: 10 Gew% Misch-Tocopherol (bezogen auf das Misch-Tocopherol-Solubilisat = 100Gew%) wird mit 90 Gew% Polysorbat 20 durch Rühren vermischt, wobei der Mischvorgang durch Erwärmen auf etwa 60°C beschleunigt wird. Das Rühren wird solange fortgesetzt, bis Klarheit erreicht ist und das Solubilisat sich in Wasser leicht lösen läßt. Für das Misch-Tocopherol empfiehlt sich eine Zusammensetzung von 91mg/g Misch-Tocopherol an α-Tocopherol, 21 mg/g an β-Tocopherol, 608 mg/g an γ-Tocopherol und 209 mg/g an δ-Tocopherol.

Sodann werden etwa 3 Gewichtsteile des Solubilisats nach Beispiel 2 erste Alternative mit etwa 7 Gewichtsteilen des Misch-Tocopherol-Solubilisats vermischt, wobei eine leichte Erwärmung auf etwa 50°C den Mischvorgang beschleunigt. Das Rühren in der Wärme wird solange ausgeführt, bis sich ein homogenes und klares Solubilisat ergeben hat. 1g dieses Produktes enthält somit etwa 70 mg Misch-Tocopherol und etwa 30 mg Ascorbinsäure. Dieses Solubilisat kann als wirksames Antioxydans zur Verbesserung der Haltbarkeit von Lebensmittel-Farbstoffen, Ölen, Kosmetika, Pharmazeutika und dergleichen eingesetzt werden.

Vergleichstests belegen, daß sich dieses Solubilisat unabhängig von den Eigenschaften des Endproduktes (hydrophil oder hydrophob) direkt und ohne Produktionszwischenschritte in diese einarbeiten läßt und einen besseren Schutz vor Oxydation bietet als die gleiche Menge an Ascorbinsäure aus einem Ascorbinsäurederivat wie beispielsweise Ascorbylpalmitat. Auch die relative Farblosigkeit des Solubilisats im Vergleich zu den Ascorbinsäurederivaten stellt einen Vorteil dar. Je nach Bedarf können beispielsweise 1 bis 10 g des Solubilisats etwa 1000 g des Endproduktes zugegeben werden.

Der ernährungsphysiologische Vorteil des Solubilisats liegt in den magensäurestabilen Micellen, die dafür sorgen, daß Ascorbinsäure (Vitamin C) und (bei Verwendung von α-Tocopherol) Vitamin E den Dünndarm verlustfrei erreichen, um dort resorbiert werden zu können. Dieser Vorteil läßt sich auch für weitere Vitamine, beispielsweise Retinol (Vitamin A) und β-Carotin nutzen und mit folgendem beispielshaften Multivitaminpräparat erreichen:

### Beispiel 5

20 Gew% α-Tocopherol werden, gegebenenfalls in der Wärme von etwa 50°C, mit 80 Gew% Polysorbat 20 bis zur Klarheit und Homogenität durch Rühren vermischt.

10 Gew% Retinol werden auf die gleiche Weise mit 90 Gew% Polysorbat 80 vermischt.
Schließlich werden 10 Gew% eines Konzentrats, das etwa 30% β-Carotin enthält (erhältlich bei La Roche), mit 90 Gew% Polysorbat 80 in entsprechender Weise zu einem rotbraunen, transparenten Solubilisat vermischt, das sich in temperiertem Wasser klar lösen läßt.
Sodann werden 85 Gew% des Ascorbinsäuresolubilisats nach Beispiels 2 erste Alternative mit 10 Gew% vorstehenden α-Tocopherolsolubilisats sowie mit 2 Gew% des vorstehenden Retinolsolubilisats und schließlich mit 3 Gew% des β-Carotinsolubilisats zweckmäßig in leichter Wärme durch Rühren miteinander vermischt, bis ein homogenes und transparentes Mischsolubilisat vorliegt. Dieses wasserlösliche Vitaminsolubilisat läßt sich in Gelatine- oder gelatinefreien Kapseln verpacken oder in wässrigen und/oder fettlöslichen Endprodukten ohne zusätzliche Verarbeitungsschritte direkt einarbeiten.

Eingebracht in Wasser und/oder klaren Frucht- oder Obstsäften ergibt das erfindungsgemäße Vitaminsolubilisat im Gegensatz zu Emulsionen oder Liposomen eine stabile und klare Lösung. Die Produktmicellen sind magensäurestabil. Die Resorption der in den Micellen befindlichen fettlöslichen Substanzen wie Vitamin A, Vitamin E und β-Carotin erfolgt im Dünndarm ohne Beteiligung von Gallensalzen und Enzymen. Daher sind die genannten Wirkstoffe in der vorliegenden micellierten Form schneller bioverfügbar.

1 Gramm des Vitaminsolubilisats der vorstehenden Zusammensetzung eingearbeitet in Lebensmittel oder eingebracht in Kapseln deckt den menschlichen Tagesbedarf an den Vitaminen A, C, E und β-Carotin. Auf diesen Umstand ist die vorstehend angegebene zahlenmäßige Zusammensetzung des Vitaminsolubilisats abgestellt. Darin kommt zum Ausdruck, daß der Tagesbedarf an Vitamin C wesentlich höher ist als derjenige an anderen Vitaminen. Es liegt jedoch im Rahmen der Erfindung, für das Vitaminsolubilisat auch andere Zusammensetzungen zu wählen und/oder auf das eine oder andere Vitamin überhaupt ganz zu verzichten, wenn der vorgesehene Verwendungszweck das Vorhandensein eines bestimmten Vitamins im Vitaminsolubilisat nicht erforderlich oder wünschenswert macht.

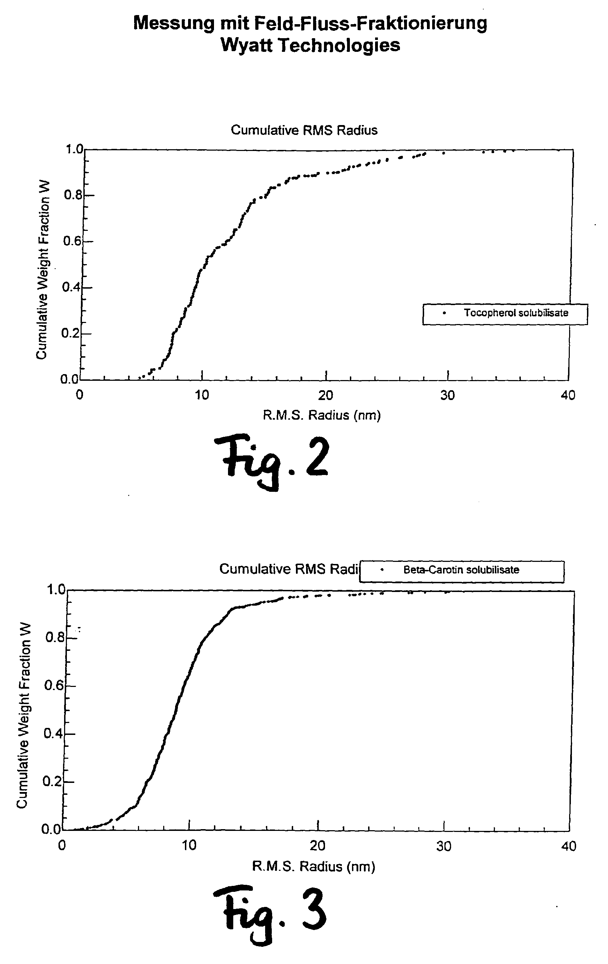

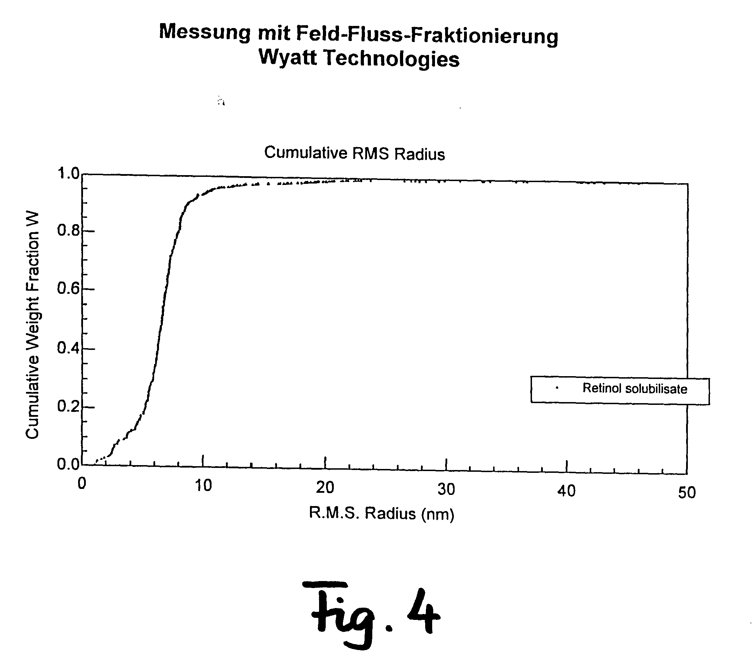

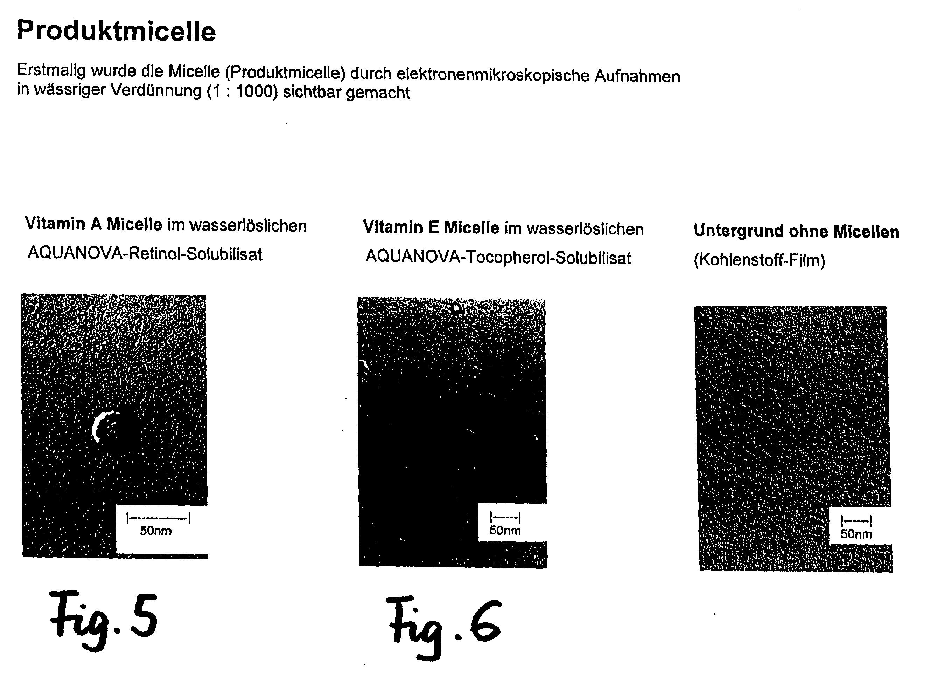

Die Figuren 2, 3 und 4 zeigen die mittleren Micellenradien des α-Tocopherol-solubilisats, des β-Carotinsolubilisats und des Retinolsolubilisats. Wie ersichtlich liegen die mittleren Micellenradien bei 10 nm und für Retinolsolubilisat sogar bei nur etwa 8 nm. Die Messungen wurden mit der Feld-Fluß-Fraktionierung der Wyatt Technologies durchgeführt. Figur 5 zeigt eine elektronenmikroskopische Aufnahme einer Vitamin A Micelle, und Figur 6 eine elektrononmikroslopische Aufnahme von Vitamin E Micellen; die Proben waren eine wässrige Verdünnung von 1 : 1000 der oben beschriebenen Solubilisate.

## Patentansprüche

1. Tocopherol-Konzentrat bestehend aus einem Solubilisat aus 10 Gew% eines Misch-Tocopherols und 90 Gew% Polysorbat 20.

2. Konzentrat nach Anspruch 1 mit 91 mg α-Tocopherol, 21 mg β-Tocopherol, 608 mg γ-Tocopherol und 209 mg δ-Tocopherol jeweilspro Gramm Mischtocopherol.

3. Verfahren zur Herstellung eines Tocopherol-Konzentrats nach Anspruch 1 oder 2, bei dem 10 Gew% Mischtocopherol mit 90 Gew% Polysorbat 20 bei etwa 60 °C vermischt und so lange gerührt wird, bis Klarheit der Mischung und leichte Löslichkeit in Wasser erreicht sind.
